# DEMANDE DE BREVET EUROPEEN

(11) **EP 2 896 430 A1**
(43) Date de publication de la demande: **22.07.2015**
(21) Numéro de dépôt: 14181593.6
(22) Date de dépôt: 20.08.2014
(51) Int. Cl.: A61Q 19/08, A61K 8/97

(54) **Utilisation cosmétique ou dermatologique d'un extrait de Quassia amara**

(30) Priorité: 23.08.2013 FR 1358164
(71) Demandeur: BASF Beauty Care Solutions France SAS, 69007 Lyon (FR)
(72) Inventeur: Cenizo, Valérie, 69008 LYON (FR); Andre, Valérie, 69420 AMPUIS (FR)
(74) Mandataire: Mendelsohn, Isabelle M. N.

(57) **Abrégé**

La présente invention concerne l'utilisation cosmétique et/ou dermatologique d'un extrait de la plante tropicale *Quassia amara* pour augmenter l'expression génique et/ou protéique du collagène. L'invention concerne également l'utilisation cosmétique et/ou dermatologique d'un tel extrait pour augmenter la fermeté de la peau et/ou des muqueuses.

L'invention concerne par ailleurs un procédé de soin cosmétique comprenant l'application par voie topique d'une composition contenant l'extrait de *Q. amara,* sur au moins une partie de la peau, pour augmenter l'expression génique et/ou protéique du collagène.

## Description

La présente invention concerne l'utilisation cosmétique et/ou dermatologique d'un extrait de plante de *Quassia amara* pour augmenter l'expression génique et/ou protéique du collagène dans la peau et/ou les muqueuses et/ou augmenter la fermeté de la peau et/ou des muqueuses.

Le collagène est une protéine constitutive de la matrice extra-cellulaire (MEC) présente en grande quantité dans les tissus des vertébrés. Il s'agit d'une large famille comprenant 29 types différents.

Parmi les différents types de collagène, on retrouve en particulier le collagène de type I dans de nombreux tissus humains tels que tendons, ligaments, cornée et peau, localisé plus précisément au niveau du derme. Le collagène de type I est le collagène majoritaire de la peau.

Le collagène fibrillaire est formé à partir des fibres de procollagène, ensuite assemblées en réseau, puis stabilisées par réticulation. C'est le collagène qui confère aux tissus leur résistance mécanique, et par voie de conséquence, participe au maintien de leur fermeté.

Au cours du vieillissement de la peau, le taux de collagène diminue globalement, ce qui entraîne une perte de fermeté. Ce phénomène est d'autant plus prononcé que la peau est entre autre soumise aux UV. On parle de vieillissement photobiologique.

Plusieurs mécanismes sont impliqués dans la diminution du taux de collagène au cours du vieillissement tissulaire: des enzymes appelées collagénases sont responsables de la dégradation des protéines de collagène. En parallèle, un mécanisme de glycation non enzymatique à l'origine de la formation de protéines glyquées appelées AGEs (Advanced Glycation End Products), dans la matrice extra-cellulaire, participe à une diminution de la fonctionnalité de la dite matrice, donc de la peau.

Le collagène est donc l'objet de nombreuses études et innovations dans le domaine de la cosmétique pour la mise au point et l'amélioration d'actifs cosmétiques visant à maintenir la fermeté de la peau.

Ainsi, des compositions cosmétiques dites à effet anti-âge ou anti-vieillissement sont connues de l'homme du métier. Certains actifs cosmétiques inhibent par exemple l'activité des collagénases, responsables de la dégradation du collagène. D'autres actifs inhibent le mécanisme de glycation du collagène.

De tels ingrédients ne permettent néanmoins que d'empêcher la dégradation du collagène et d'en maintenir le taux dans la peau mais ils ne permettent pas de l'augmenter ni d' augmenter la fermeté de la peau.

Le but de la présente invention est de fournir un nouvel ingrédient cosmétique et/ou dermatologique permettant d'augmenter l'expression génique et/ou protéique du collagène, et/ou d'augmenter la fermeté de la peau et/ou des muqueuses.

Cet ingrédient cosmétique et/ou dermatologique doit être topiquement acceptable, facilement formulable et/ou utilisable dans une composition cosmétique et/ou dermatologique et administrable par voie topique, sur tout ou partie de la peau du corps humain et/ou des muqueuses humaines.

L'invention a enfin pour but de fournir un procédé de soin cosmétique par application par voie topique de la composition contenant l'ingrédient cosmétique pour augmenter l'expression génique et/ou protéique du collagène dans la peau et/ou les muqueuses et/ou pour augmenter la fermeté de la peau et/ou des muqueuses.

De manière surprenante, les inventeurs ont découvert qu'un extrait de la plante *Q. amara* augmentait l'expression génique et/ou protéique du collagène, préférentiellement l'expression protéique du collagène, et encore préférentiellement du collagène de type I.

Outre cette propriété, l'extrait de *Q*. *amara* présente l'avantage d'être topiquement acceptable, facilement formulable et/ou utilisable dans une composition cosmétique et/ou dermatologique et administrable par voie topique, sur tout ou partie de la peau du corps humain et/ou des muqueuses humaines

*Quassia amara* est un arbuste tropical de la famille des Simaroubaceae, de 2 à 5 mètres de hauteur originaire de Guyane, que l'on trouve aujourd'hui principalement en Amérique centrale et du Sud. Il est communément désigné sous le nom de Quassia du Surinam ou amargo, souvent confondu avec le Quassia de Jamaique.

L'écorce et les racines sont connues pour être utilisées dans les cas d'anémie, anorexie, ou encore les cas de dysfonctionnements gastriques.

*Quassia amara (Q. amara)* est utilisée par voie orale en Europe et aux Etats-Unis en tant qu'herbe médicinale et est connue pour stimuler des organes internes tels que foie et vésicule biliaire.

La plante présente une amertume importante, supérieure à celle de la quinine, en faisant un puissant tonique non astringent. Elle est conseillée et utilisée aux Etats-Unis pour stimuler la salive et les sucs gastriques.

Le bois de *Q. amara* est aussi connu pour ses propriétés anti-microbiennes.

Il était déjà connu des utilisations cosmétiques d'extraits de *Q. amara.* Ainsi, le brevet US8293291 décrit une méthode de diminution de l'apparition des rides d'expression du visage par l'intermédiaire d'une composition contenant un ou plusieurs actifs végétaux, parmi lesquels un extrait de *Q. amara,* par un effet mécanique sur les muscles du visage.

L'extrait de *Q. amara* est en effet décrit comme un inhibiteur du métabolisme de l'acétylcholine, neurotransmetteur responsable du relargage de calcium dans les fibres musculaires, entrainant leur contraction. Cette inhibition empêche la contraction des muscles, avec pour conséquence un effet « botox-like ».

Par ailleurs, l'invention décrite dans la demande WO 2007/048985 déposée par la demanderesse décrit l'utilisation d'un extrait de bois de *Q. amara* stimulant l'expression des Lysyl Oxidases (LOX), protéines impliquées dans la stabilisation de la matrice extra-cellulaire (MEC).

Toutefois, aucune des utilisations connues d'un extrait de *Q. amara* ne décrit ni suggère ses propriétés d'augmentation de l'expression génique et/ou protéique du collagène, et donc de prédire la présente invention.

Ainsi, la présente invention a pour objet l'utilisation cosmétique et/ou dermatologique, avantageusement par voie topique, d'un extrait de *Q. amara* pour augmenter l'expression génique et/ou protéique du collagène, en particulier dans la peau et/ou les muqueuses, et/ou pour augmenter la fermeté de la peau et/ou des muqueuses.

Au sens de la présente invention, on entend par « utilisation et/ou composition cosmétique », une utilisation et/ou composition non pharmaceutique, c'est-à-dire qui n'est pas destinée à un usage thérapeutique et est appliquée sur une partie du corps dite saine, en particulier sur une zone de la peau et/ou des muqueuses dite saine.

Au sens de la présente invention on entend par «peau saine» ou «muqueuse saine», une zone de peau ou de muqueuse sur laquelle est appliquée l'extrait selon l'invention et dite « non pathologique » par un dermatologue, c'est à dire ne présentant pas d'infection, de cicatrice, de maladie ou d'affection cutanée telle que candidose, impétigo, psoriasis, eczéma, acné ou dermatite, ou de plaies ou de blessures.

Au sens de la présente invention, on entend par « collagène », les protéines de collagène de type I, III, IV, V, VI, VII, XII, XIII, XIV, XVI, XVII, XXIV, XXIX, présentes dans la peau et/ou les muqueuses. Avantageusement, il s'agit des protéines de collagène de type I, III, IV, V et VII, préférentiellement des protéines de collagène de type I, de type III et de type V et encore préférentiellement la protéine de collagène de type I. De manière préférentiellement, le collagène est le collagène humain, en particulier de la peau et/ou des muqueuses humaines. Avantageusement selon l'invention, on entend par « collagène de type III », la protéine de collagène présente dans la peau, et préférentiellement dans la matrice extra-cellulaire et/ou au niveau de la paroi des vaisseaux sanguins cutanés.

Au sens de la présente invention, on entend par protéine de collagène de type I, la protéine de collagène présente dans la peau, la cornée, les tendons, les ligaments, les os, encore préférentiellement dans la peau.

La présente invention concerne ainsi l'utilisation cosmétique et/ou dermatologique, avantageusement par voie topique, d'un extrait de *Q. amara,* en particulier comme ingrédient actif, pour augmenter l'expression génique et/ou protéique du collagène, préférentiellement du collagène de type I, et/ou pour augmenter la fermeté de la peau et/ou les muqueuses.

Au sens de la présente invention, on entend par « l'expression du collagène », l'expression génique du collagène, c'est-à-dire l'expression des ARNm (ARN messagers) et/ou l'expression protéique du collagène.

Préférentiellement, il s'agit de l'expression protéique du collagène.

Au sens de la présente invention, on entend par « augmenter l'expression génique et/ou protéique du collagène », une augmentation du niveau d'expression génique et/ou protéique du collagène d'au moins 20 %, préférentiellement d'au moins 40 % et encore plus préférentiellement d'au moins 50 %, par rapport au niveau d'expression génique et/ou protéique du collagène mesuré en l'absence de l'extrait de *Q*. *amara* selon l'invention. Préférentiellement, l'augmentation de l'expression est une augmentation de l'expression protéique du collagène et encore préférentiellement du collagène de type I.

Dans un mode préférentiel de l'invention, l'extrait de la plante *Q. amara* selon l'invention est donc utilisé pour augmenter l'expression protéique du collagène de type I au niveau de la peau.

Selon un mode avantageux de l'invention, il s'agit d'une augmentation par rapport au niveau d'expression génique et/ou protéique du collagène mesuré dans des cellules de fibroblastes dermiques cultivées *in vitro* en l'absence de l'extrait de *Q*. *amara* selon l'invention.

Dans un mode préférentiel de l'invention, il s'agit d'une augmentation du niveau d'expression protéique du collagène de type I d'au moins 50 % par rapport au niveau d'expression protéique mesuré dans des cellules de fibroblastes dermiques cultivées *in vitro* en l'absence de l'extrait selon le protocole tel que décrit par exemple dans l'exemple 2.

Avantageusement, la mesure de l'augmentation de l'expression protéique est effectuée par mesure *in vitro,* préférentiellement par mesure par microscopie confocale après immunomarquage par exemple selon la méthode telle que présentée en exemple 2.

Au sens de la présente invention, on entend par « immunomarquage », la technique consistant à fixer un anticorps spécifique d'une protéine donnée de collagène, préférentiellement la protéine de collagène de type I, sur la dite protéine, en vue d'une révélation par observation microscopique. Avantageusement, la révélation se fait par microscopie confocale.

Au sens de la présente invention, on entend d'un point de vue cosmétique par « augmenter la fermeté de la peau et/ou des muqueuses », une augmentation à des fins esthétiques de la fermeté de la peau et/ou des muqueuses qui ont perdu de la fermeté notamment sous l'effet de facteurs intrinsèques tels que le vieillissement tissulaire de la peau et/ou des muqueuses, c'est-à-dire le vieillissement chronoinduit, que l'on retrouve par exemple chez les peaux dites matures c'est-à-dire les peaux de personnes de plus de 40ans, le stress cellulaire, les variations physiologiques non pathologiques telles que un changement de régime alimentaire, les variations hormonales, en particulier durant la puberté, la grossesse, la ménopause, l'andropause. Cette perte de fermeté peut également apparaitre sous l'effet de facteurs extrinsèques tels que les agents agressifs de l'environnement comme par exemple les radiations UV, la pollution, les fumées, le tabac, les toxines, les agressions climatiques et/ou mécaniques. Il peut notamment s'agir du soin et/ou traitement cosmétique de l'aspect inesthétique et/ou inconfortable des vergetures.

Au sens de la présente invention, on entend d'un point de vue dermatologique par « augmenter la fermeté de la peau et/ou des muqueuses », une augmentation à des fins thérapeutiques de la fermeté de la peau et/ou des muqueuses qui ont perdu de la fermeté sous l'effet de pathologies de la peau et/ou des muqueuses comme par exemple la couperose, les télangiectasies. L'augmentation de la fermeté peut être mesurée selon les méthodes classiques notamment par mesure *in vivo* par la méthode de projections de frange à l'aide d'un cutomètre, d'un densiscore, d'un torquemètre ou encore d'un dynaskin associé à un dermatop.

Selon l'invention, on désigne par « muqueuse(s) », la muqueuse oculaire, la muqueuse vaginale, la muqueuse uro-génitale et/ou la muqueuse buccale, notamment buccale labiale et/ou la muqueuse gingivale, préférentiellement, les muqueuses oculaires et/ou buccales, etencore préférentiellement, la muqueuse labiale et/ou oculaire.

L'extrait de *Q. amara* selon l'invention est un extrait cosmétique et/ou dermatologique topiquement acceptable.

Au sens de la présente invention, on entend par « cosmétique et/ou dermatologique topiquement acceptable », un ingrédient adapté à une application par voie topique, non toxique, non irritant pour la peau et/ou les muqueuses, n'induisant pas de réponse allergique, qui n'est pas instable sur le plan chimique.

Au sens de la présente invention, on entend par « application par voie topique » d'un ingrédient, l'application locale directe et/ou la vaporisation de l'ingrédient sur la surface de la peau et/ou des muqueuses.

L'extrait de *Q. amara* selon l'invention peut être tout extrait de la plante choisi parmi la plante entière, le bois, les racines, le rhizome, l'écorce, les fleurs, les pétales, les sépales, les graines, les fruits, la pellicule des fruits, les parties aériennes, en particulier la tige, les feuilles et leurs mélanges. Avantageusement, l'extrait de Q. *amara* selon l'invention est obtenu par extraction du bois de *Q. amara,* encore préférentiellement du bois sans écorce. De manière avantageuse, l'extrait selon l'invention est extrait du bois seulement, sans écorce.

La partie de la plante est préférentiellement séchée et/ou broyée avant extraction.

L'extrait de *Q. amara* selon l'invention peut être obtenu par les méthodes d'extraction de végétaux connues dans le domaine, par exemple par macération d'au moins une partie de la plante de préférence entre 1 et 10% (p/p) de matière sèche dans un solvant ou un mélange de solvants, de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol, butylène glycol ou autres glycols, tel que xylitol etc.) de 100/0 à 0/100 (v/v).

Les extraits obtenus sont ensuite de préférence centrifugés et/ou filtrés et/ou distillés afin de récupérer la fraction soluble active (extrait brut). Des étapes supplémentaires de décoloration et/ou désodorisation peuvent être effectuées sur l'extrait à n'importe quel stade de l'extraction et selon les techniques connues par l'Homme du métier.

Selon un mode préférentiel, l'extrait est obtenu par extraction aqueuse, préférentiellement dans l'eau seulement. L'extraction peut être réalisée durant une période allant de 1 heures à 20 heures, préférentiellement durant une période allant de 2 heures à 16 heures. Préférentiellement encore, la dite extraction est réalisée durant une période de 2 heures.

L'extraction peut être réalisée à une température allant de 2 à 25 °C. Préférentiellement, la dite extraction est effectuée à température ambiante, c'est-à-dire à 20 °C.

L'extraction peut être réalisée par macération, par broyage à l'aide d'un broyeur à billes, broyage au mortier, broyage aux ultrasons, broyage à l'aide d'un mixeur. Préférentiellement, l'extraction est réalisée par macération.

L'extrait de *Q. amara* peut être obtenu par extraction d'une quantité de 1 % à 10 % en poids de matière sèche d'au moins une partie de la plante par rapport au poids total du mélange constitué du solvant et de la partie de plante (p/p). Préférentiellement, l'extrait est obtenu par extraction d'une quantité de 5 % en poids de matière sèche d'au moins une partie de la plante, par rapport au poids total du mélange constitué du solvant, préférentiellement l'eau, et de la plante (p/p).

Dans un mode de réalisation préférentiel de l'invention, l'extrait est un extrait aqueux obtenu par macération durant une période de 2 heures à température ambiante, donc à 20 °C, de 5 % en poids de matière sèche de bois par rapport au poids total du mélange constitué de l'eau et du bois, en particulier selon le protocole décrit dans l'exemple 1a).

Dans un autre mode préférentiel de l'invention, l'extrait de *Q. amara* est un extrait aqueux obtenu par macération aqueuse durant une période de 16 heures à 4 °C, de 5 % en poids de matière sèche de bois par rapport au poids total du mélange constitué de l'eau et du bois, en particulier selon le protocole décrit dans l'exemple 1b).

Dans encore un autre mode préférentiel de l'invention, l'extrait de *Q. amara* est obtenu par macération dans un mélange eau/butylène glycol (75/25 ; v/v) de 5 % en poids de matière sèche de bois par rapport au poids total du mélange constitué du mélange eau/butylène glycol et du bois, durant une période de 2 heures à température ambiante, en particulier selon le protocole décrit dans l'exemple 1c).

Dans un mode alternatif de l'invention, l'extrait est un extrait aqueux de feuille obtenu par broyage, à température ambiante, de 5 % en poids de matière sèche de feuille par rapport au poids total du mélange constitué de l'eau et des feuilles, en particulier selon le protocole décrit dans l'exemple 1d).

L'extrait obtenu selon l'invention est de préférence centrifugé pour récupérer la fraction soluble. Préférentiellement, le surnageant obtenu est ensuite filtré (seuil de coupure de 0,45 µm) par des filtres connus de l'homme du métier.

L'extrait selon l'invention peut être ensuite concentré par évaporation du solvant ou séché par exemple par lyophilisation ou par atomisation.

Dans un mode particulier de réalisation de l'invention notamment pour son utilisation en dermatologie, l'extrait de *Q. amara* obtenu selon l'invention peut être stérilisé.

L'extrait de *Q*. *amara* selon l'invention est ensuite préférentiellement dissous dans un solvant notamment polaire, en particulier choisi parmi l'eau, un alcool, un polyol, un glycol, tel que le pentylène glycol et/ou le butylène glycol, ou un de leurs mélanges, avantageusement il s'agit d'un mélange d'eau et de glycol tels que le butylène glycol et/ou le pentylène glycol.

Selon un mode avantageux, l'extrait est ainsi solubilisé dans un véhicule aqueux, préférentiellement de l'eau. L'extrait est ensuite utilisé conformément à la présente invention, éventuellement après filtration.

Selon l'invention, l'extrait de *Q. amara* peut être utilisé seul ou dans une composition cosmétique et/ou dermatologique destinée à une application par voie topique, en particulier sur la peau et/ou les muqueuses du corps humain. Selon un mode préférentiel, l'extrait est appliqué sur au moins une zone de peau et/ou de muqueuse choisie parmi le cou, le décolleté, le ventre, les bras, les cuisses, les hanches, les fesses, la taille et/ou le visage, et préférentiellement le contour des yeux, les lèvres.

La présente invention concerne donc l'utilisation d'une composition cosmétique et/ou dermatologique qui contient un extrait de *Q*. *amara* selon l'invention, préférentiellement un extrait aqueux de bois de *Q. amara,* pour augmenter l'expression génique et/ou protéique du collagène, préférentiellement du collagène de type I, et/ou pour augmenter la fermeté de la peau et/ou des muqueuses.

Dans le cas des applications dermatologiques, l'extrait de *Q. amara* selon l'invention et/ou la composition dermatologique selon l'invention est préférentiellement utilisée pour le soin et/ou le traitement dermatologique des pathologies impliquant une perte d'expression génique et/ou protéique du collagène, notamment du collagène de type I, et/ou une perte de fermeté pathologiques, telles que dans le cas des pathologies de la peau et/ou des muqueuses, telles que la couperose, des télangiectasies.

La composition cosmétique et/ou dermatologique selon l'invention avantageusement contient l'extrait selon l'invention à une concentration comprise entre 1.10⁻⁴ % et 10 % en poids par rapport au poids total de la composition. Avantageusement encore, la composition selon l'invention contient l'extrait selon l'invention à une concentration comprise entre 1.10⁻³ % et 3 %, encore préférentiellement entre 1.10⁻² % et 1 % en poids par rapport au poids total de la composition.

Par ailleurs, la composition cosmétique et/ou dermatologique contient au moins un excipient cosmétique et/ou dermatologique topiquement acceptable.

Avantageusement, le ou lesdits excipients sont choisis parmi au moins un des groupes constitués par les conservateurs, les émollients, les émulsifiants, les tensioactifs, les hydratants, les épaississants, les agents de texture, les agents filmogènes, les pigments, les stabilisants, les solubilisants, les colorants, les parfums.

Avantageusement encore, le ou les excipients sont choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les gommes de xanthane, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

La composition cosmétique et/ou dermatologique de l'invention peut être choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; une mousse ; un patch ; un produit anhydre, de préférence liquide, pâteux ou solide, par exemple sous forme de poudres de maquillage, de bâtonnet ou de stick, notamment sous forme de rouge à lèvre.

De façon avantageuse, la composition de l'invention est une crème ou un sérum, destinée à être appliquée sur des parties spécifiques du corps tels que le cou, le décolleté, le ventre, les bras, les cuisses, les hanches, les fesses, la taille et/ou le visage, et préférentiellement le contour des yeux, les lèvres.

En outre, la composition cosmétique et/ou dermatologique de la présente invention peut contenir un ou plusieurs ingrédients actifs cosmétiques, conduisant à un effet complémentaire et/ou un effet de synergie avec l'extrait de *Q. amara* selon l'invention.

Il peut s'agir par exemple d'ingrédients anti-rides, agents stimulant l'activité ou la prolifération des fibroblastes, agents stimulants les molécules de la matrice extra-cellulaire.

Il peut s'agir par ailleurs d'ingrédients actifs anti-âges et/ou agents tenseurs pour un effet de synergie avec l'extrait de *Q. amara.* Avantageusement, il s'agit d'ingrédients actifs augmentant l'expression génique et/ou protéique du collagène ou d'ingrédients actifs empêchant la dégradation du dit collagène tels que le rétinol, la vitamine C, un extrait de *Davilla rugosa* commercialisé sous le nom de Collguard par BASF Beauty Care Solutions, un extrait de graine d'*Hibiscus abelmoschus* commercialisé sous le nom de Linefactor, un peptide commercialisé sous le nom de Dermican par la demanderesse ou encore les produits commercialisés sous les noms de Matrixyl, matrixyl 3000 et Regestril par la société Sederma.

Les agents tenseurs utilisables dans l'invention peuvent être choisis parmi les polymères synthétiques, tels que les latex de polyuréthanne ou les latex acryliques, les polymères d'origine naturelle, notamment les polyholosides sous forme d'amidon ou sous forme de carraghénanes, alginates, agars, gellanes, polymères cellulosiques et pectines; les protéines et hydrolysats de protéines végétales de soja ; les silicates mixtes ; les microparticules de cire ; les particules colloïdales de charge inorganique choisies par exemple parmi la silice, les composites silice-alumine ; ainsi que leurs mélanges.

Il peut enfin s'agir d'ingrédients cosmétiques comme par exemple des agents antimicrobiens, agents anti-radicalaires, agents apaisants, calmants ou relaxants, agents agissant sur la microcirculation pour améliorer l'éclat du teint, en particulier du visage, agents cicatrisants ou agents amincissants. Avantageusement la composition cosmétique et/ou dermatologique de la présente invention contient également un ou plusieurs agents tenseurs et/ou un ou plusieurs agents antimicrobiens et/ou un ou plusieurs agents antiradicalaires et/ou un ou plusieurs agents apaisants et/ou un ou plusieurs agents amincissants et/ou un ou plusieurs agents actifs sur la microcirculation.

Parmi les agents antimicrobiens pouvant être associés à l'extrait de *Q*. *amara* dans la présente invention, on peut citer le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octoxyglycérine, l'octanoylglycine, le caprylyl glycol, l'acide 10-hydroxy-2-décanoïque, le farnesol, les phytosphingosines et leurs mélanges.

Les agents anti-radicalaires peuvent être la vitamine C et ses dérivés dont le glucoside d'ascorbyle, les phénols et polyphénols, en particulier les tannins, l'acide ellagique et l'acide tannique; l'épigallocatéchine et les extraits naturels en contenant, en particulier les extraits de thé vert; les anthocyanes; les acides phénols, les stilbènes; des actifs piégeurs de composés aromatiques mono- ou polycycliques les tannins tels que l'acide ellagique et les dérivés indoles et/ou des actifs piégeurs de métaux lourds tels que l'EDTA, des actifs anti-radicalaires tels que la vitamine E et ses dérivés tels que l'acétate de tocophéryle ; les bioflavonoïdes; la coenzyme Q10 ou ubiquinone.

Comme agents apaisants entrant dans la composition de l'invention, on peut utiliser les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de *Pueraria lobata* commercialisé sous le nom Inhipase™ par la demanderesse, les extraits de Theobroma cacao.

Les ingrédients actifs agissant sur la microcirculation, vasoprotecteurs ou vasodilatateurs, peuvent être choisis parmi les flavonoïdes, les ruscogénines, les nicotinates, les huiles essentielles.

Les actifs amincissants peuvent être notamment choisis parmi les agents inhibiteurs de la lipoprotéine lipase tels que ceux décrits dans le brevet US2003086949 (Coletica) et en particulier un extrait de liane du Pérou (*Uncaria tomentosa*); les actifs drainants, notamment l'hesperitine laurate (Flavagrum™), ou quercitine caprylate (Flavenger™); les agents inhibiteurs de l'enzyme phosphodiestarase, les agents activateurs de l'adenylate cyclase, l'AMPc et/ou les actifs capable de piéger la spermine et/ou la spermidine. On peut citer un extrait de racine de *Coleus Forskohlii,* un extrait de *cecropia obtusa,* d'*Uva lactuca,* la caféine, la forskoline, la théophylline, la théobromine et/ou leurs dérivés, un produit de kappa carraghénanes hydrolysé dénommé Slimexcess™ commercialisé par la demanderesse et/ou leurs mélanges.

L'invention concerne enfin un procédé de soin cosmétique comprenant l'application par voie topique de l'extrait de *Q*. *amara* selon l'invention ou d'une composition cosmétique selon l'invention sur au moins une zone de la peau du corps, en particulier du corps humain, et/ou des muqueuses, en particulier des muqueuses humaines, pour augmenter l'expression génique et/ou protéique du collagène, en particulier du collagène de type I, et/ou pour augmenter la fermeté de la peau et/ou des muqueuses.

Dans un mode de réalisation de l'invention, le procédé de soin cosmétique comprend l'application par voie topique de l'extrait de *Q. amara* selon l'invention notamment sous la forme d'une composition cosmétique selon l'invention. Préférentiellement, le procédé de soin cosmétique consiste en l'application par voie topique de l'ingrédient actif contenant un extrait aqueux de 5 % (en poids par rapport au poids total de l'ingrédient) de bois de *Q. amara* (p/p) selon l'exemple 3.

Dans un autre mode de réalisation de l'invention, le procédé de soin cosmétique comprend l'application par voie topique de la composition cosmétique selon l'invention, contenant l'extrait selon l'invention à une concentration comprise entre 1.10⁻⁴ % et 10 % en poids par rapport au poids total de la composition. Avantageusement encore, la composition selon l'invention contient l'extrait selon l'invention à une concentration comprise entre 1.10⁻³ % et 3 %, encore préférentiellement entre 1.10⁻² % et 1 % en poids par rapport au poids total de la composition.

De préférence, le procédé de soin cosmétique consiste en l'application par voie topique de l'extrait de *Q*. *amara* selon l'invention ou de la composition cosmétique contenant ledit extrait, sur la zone de peau et/ou muqueuses choisie parmi le cou, le décolleté, le ventre, les bras, les cuisses, les hanches, les fesses, la taille et/ou le visage, et préférentiellement le contour des yeux et/ou les lèvres.

Préférentiellement, ledit procédé selon l'invention est utilisé pour augmenter l'expression protéique du collagène de type I.

Le procédé de soin cosmétique est également utilisé pour augmenter la fermeté de la peau et/ou des muqueuses.

L'invention sera mieux comprise à la lecture de la description des figures et des exemples qui suivent.

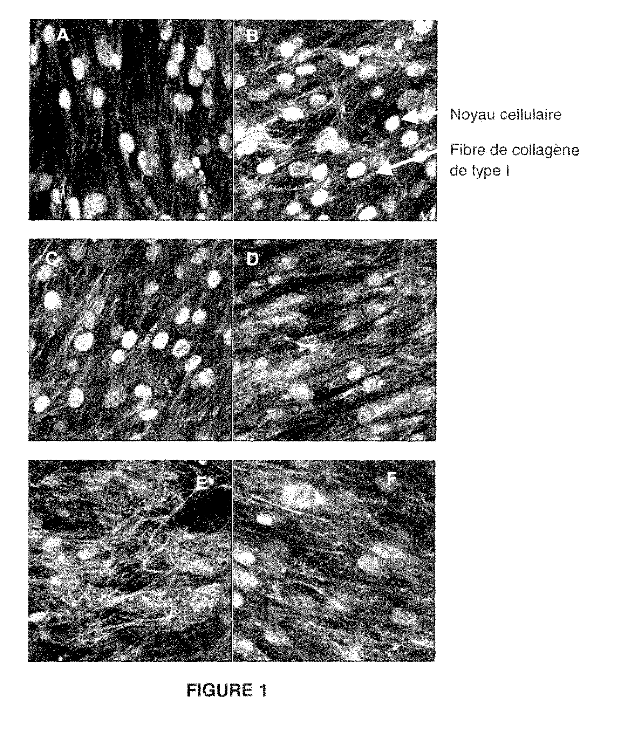

La figure 1 représente la visualisation des propriétés de l'extrait selon l'invention sur l'augmentation de l'expression protéique du collagène de type I par microscopie confocale lors de l'expérience décrite selon l'exemple 2.
A. Milieu de culture témoin non traité;
B. Milieu de culture traité avec de la vitamine C (50 µg/mL);
C. Milieu de culture témoin traité avec le TGFβ1 (Transforming Growth Factor β1) (1 ng/mL) ;
D. Milieu de culture témoin traité avec du rétinaldéhyde (1 µM) ;
E. Milieu de culture traité avec un extrait de *Q*. *amara* obtenu selon l'exemple 1a) à 0,01 % final (p/p);
F. Milieu de culture traité avec un extrait de *Q. amara* obtenu selon l'exemple 1a) à 0,05 % final (p/p).

Des exemples faisant référence à la description de l'invention sont présentés ci-après. Ces exemples sont donnés à titre d'illustration et ne sauraient limiter en aucun cas la portée de l'invention. Chacun des exemples a une portée générale. Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

### Exemple 1 : Différents modes de réalisation de l'invention pour l'obtention d'un extrait de Q. amara selon l'invention.

### a) Obtention d'un extrait de bois de Q. amara selon l'invention

Cinq pour cent de bois sans écorce, en poids de matière sèche par rapport au poids total du mélange constitué de l'eau et du bois (p/p), de la plante *Q*. *amara* ont été mis à macérer dans l'eau, l'eau étant ici l'unique solvant, durant une période de 2 heures à température ambiante, c'est-à-dire à 20 °C. Après macération, l'extrait brut obtenu a été centrifugé 10 min (8000 tour par min) puis le surnageant récupéré et filtré (seuil de coupure de 0,45 µm).

L'extrait de bois à 5 % en poids a ensuite été testé sur l'augmentation de l'expression protéique du collagène de type I selon l'exemple 2 ci-après.

### b) Obtention d'un extrait de bois de Q. amara selon l'invention.

Cinq pour cent de bois sans écorce en poids de matière sèche par rapport au poids total du mélange constitué de l'eau et du bois (p/p) de la plante *Q*. *amara* ont été mis à macérer à une température de 4 °C durant une période de 16 heures dans de l'eau. L'extrait brut a ensuite été centrifugé pendant 10 min (8000 tour par min) puis le surnageant filtré (0,45 µm).

### c) Obtention d'un extrait de bois de Q. amara selon l'invention.

Cinq pour cent de bois en poids de matière sèche par rapport au poids total du mélange constitué de l'eau et du bois (p/p) de la plante *Q. amara* ont été mis à macérer à température ambiante, ici 20 °C, durant une période de 2 heures, dans un mélange eau/butylène glycol (75/25 ; v/v). L'extrait brut a ensuite été centrifugé pendant 10 min (8000 tour par min) puis le surnageant filtré (0,45 µm).

### d) Obtention d'un extrait de feuilles de Q. amara selon l'invention

Un extrait de feuilles a été obtenu par broyage dans l'eau de 5 % de feuilles en poids de matière sèche par rapport au poids total du mélange constitué de l'eau et des feuilles (p/p). Le broyage a été effectué à température ambiante, c'est à dire à 20 °C. L'extrait broyé a ensuite été centrifugé pendant 10 min (8000 tour par min) puis le surnageant filtré (0,45 µm).

### Exemple 2 : Mise en évidence des propriétés de l'extrait selon l'invention sur l'augmentation de l'expression protéique du collagène de type I dans des cellules de fibroblastes dermiques cultivées in vitro.

### Matériel et méthodes :

Un extrait de bois de *Q*. *amara* à 5 % (p/p) obtenu selon l'exemple 1a), a été testé sur des cellules de fibroblastes dermiques cultivées *in vitro.* L'extrait a été testé à des concentrations finales de 0,01 % et 0,05 % en volume dans le milieu de culture. Préférentiellement, la concentration finale était de 0,01 % (v/v).

Précisément, des fibroblastes dermiques de l'abdomen d'un donneur de 61 ans ont été ensemencés en chambre de culture (8 puits) et amplifiés en milieu de culture FGM (Fibroblast Growth Media) contenant un antibiotique, la normocine, durant 3 ou 4 jours jusqu'à confluence, stade cellulaire pour lequel 100 % des cellules cultivées adhèrent entre elles, formant un tapis cellulaire homogène.

Les cellules confluentes ont alors été traitées dans ce même milieu de culture durant 3 jours avec un extrait de bois de *Q*. *amara* à une concentration finale dans le milieu de culture de 0,01 % ou 0,05 % (v/v) (Tableau 1). D'autres principes actifs témoins ont été testés (Tableau 1) : vitamine C (50 µg/mL) ou rétinaldéhyde (1 µM) ou TGFβ1 (1 ng/mL) (Transforming Growth Factor β1).

Les milieux de culture (FGM) contenant les cellules de fibroblastes dermiques traitées par un actif ou les milieux de culture non traités contenaient par ailleurs 5 µg/mL de vitamine C, permettant au collagène nouvellement synthétisé d'être sécrété hors de la cellule.

Après 3 jours d'incubation, les cellules ont été rincées avec un tampon Phosphate Buffer Saline (PBS) contenant du calcium, du magnésium et des antibiotiques puis fixées en méthanol froid durant 10 minutes en vue de réaliser un immunomarquage.

Un anticorps anti-collagène de type I a été ajouté au milieu en vue de réaliser un immunomarquage.

L'augmentation de l'expression protéique du collagène de type I a été observée par microscopie confocale (LSM 700, Zeiss, LePecq, France) et quantifiée par analyse d'image (Figure 1).

L'expression protéique du collagène de type I a été calculée en ratio par rapport à une expression théorique égale à 1 dans le cas de cellules de fibroblastes non traitées (NT). L'expression protéique moyenne était obtenue par la moyenne de 4 essais distincts (Tableau 1).

### Résultats :

Les résultats sont présentés dans le tableau 1 ci-après et dans la figure 1.

**Tableau 1: Mesure de l'augmentation de l'expression protéique du collagène de type I dans des cellules de fibroblastes dermiques cultivées in vitro en présence de différents ingrédients testés.**

| **Ingrédients testés** | **Augmentation moyenne** | **Ecart-type** |
|---|---|---|
| Non Traité (NT) | 1,00 | 0,75 |
| Rétinaldéhyde (1 µM) | 2,06 | 0,31 |
| Vitamine C (50 µg/mL) | 2,31 | 0,88 |
| TGFβ (1 ng/mL) | 1,42 | 0,93 |
| *Q. amara* (0,01 %) | 2,35 | 0,48 |
| *Q. amara* (0,05 %) | 2,28 | 0,56 |

### Discussions :

Les résultats obtenus avec les témoins positifs habituels de stimulation d'expression protéique du collagène de type I ont permis de valider l'expérience.

De manière surprenante, l'extrait aqueux de bois de *Q. amara* à une dose finale de 0,05 % (v/v) a permis de plus que doubler l'expression protéique du collagène de type I (2,28 fois), en comparaison de l'expression protéique quantifiée dans les cellules non traitées.

Lorsque la concentration de l'extrait de bois de *Q. amara* était de 0,01 %(v/v), l'augmentation de l'expression protéique était de 2,35 fois celle obtenue dans les cellules non traitées.

### Conclusion :

Une augmentation de l'expression protéique du collagène de type I a été observée dans les cellules de fibroblastes dermiques cultivées *in vitro* traitées avec un extrait de bois de *Q*. *amara,* particulièrement avec une dose finale du dit extrait dans le milieu de culture de 0,01 % (v/v).

### Exemple 3 : Exemple d'un ingrédient cosmétique et/ou dermatologique contenant l'extrait de bois de Q. amara selon l'invention.

On procède selon les méthodes connues de l'homme de l'art pour mélanger ensemble les différents ingrédients selon la présente invention.

L'extrait de bois de *Q. amara* est celui obtenu selon l'exemple 1. a). Les % sont des pourcentages en poids par rapport au poids total de la composition.

| | |
|---|---|
| Extrait de bois de *Q. amara* | 5 % |
| Pentylène glycol | 5 % |
| Butylène glycol | 20 % |
| Keltrol (gomme de xanthane) | 0,5 % |
| Eau | Qsp 100 |

### Exemple 4 : Exemple d'une composition cosmétique contenant l'extrait selon l'invention.

La composition ci-après est préparée selon des méthodes connues de l'homme du métier, en particulier s'agissant des différentes phases à mélanger entre elles. *L'ingrédient cosmétique est préparé selon l'exemple 3 précédent. Les quantités indiquées sont en pourcentage en poids par rapport au poids total de la composition.

| | |
|---|---|
| Ingrédient cosmétique* | 3,00 |
| Gomme de xanthane | 0,50 |
| EDTA | 0,05 |
| Steareth-2 | 2,00 |
| Steareth-21 | 2,50 |
| Alcool cetearyl | 1,00 |
| Caprylate de propylheptyl | 15,00 |
| Hydroxyde de sodium (30 % en solution) | 0,10 |
| Mélange de phenoxyéthanol, chlorphenesin, acide benzoique, butylène glycol, acide sorbique (Germazide™ PBS) | 1,25 |
| Mélange de polyacrylate-X, d'isohexadecane et de polysorbate 60 | |
| (Sepigel™ SMS 60) | 4,00 |
| Eau | Qsp 100 |

### Exemple 5 : Exemple d'une composition dermatologique sous forme de pommade contenant l'extrait selon l'invention.

La composition ci-après est préparée selon des méthodes connues de l'homme du métier, en particulier s'agissant des différentes phases à mélanger entre elles. Les quantités indiquées sont en pourcentage en poids par rapport au poids total de la composition.

| | |
|---|---|
| Ingrédient * | 3,00 |
| Excipient : | |
| Polyéthylène basse densité | 5,50 |
| Parrafine liquide | Qsp 100 |

| | |
|---|---|
| * L'ingrédient est préparé selon l'exemple 3 précédent. L'ingrédient selon l'invention est stérilisé puis séché avant d'être incorporé dans la composition sous forme de pommade. | |

## Revendications

1. Utilisation cosmétique par voie topique d'un extrait de *Quassia amara* pour augmenter l'expression génique et/ou protéique du collagène.

2. Utilisation selon la revendication 1, **caractérisée en ce que** l'extrait de *Quassia amara* est obtenu par extraction aqueuse.

3. Utilisation selon l'une quelconque des revendications 1 ou 2, **caractérisée en ce que** l'extrait de *Quassia amara* est obtenu à partir de bois, avantageusement sans écorce.

4. Utilisation selon l'une quelconque des revendications 1 à 3, pour augmenter la fermeté de la peau et/ou des muqueuses.

5. Utilisation selon l'une quelconque des revendications 1 à 4, pour augmenter l'expression génique et/ou protéique du collagène de type I.

6. Utilisation selon l'une quelconque des revendications 1 à 5, **caractérisée en ce que** l'extrait de *Quassia amara* est obtenu par extraction d'une quantité de 1 à 10 % en poids de matière sèche d'au moins une partie de la plante *Quassia amara* par rapport au poids total d'un mélange solvant/plante, avantageusement de 5 % en poids.

7. Utilisation selon l'une quelconque des revendications 1 à 6, **caractérisée en ce que** l'extrait de *Quassia amara* est appliqué par voie topique sur la peau et/ou les muqueuses du corps humain.

8. Utilisation selon l'une quelconque des revendications 1 à 7, **caractérisée en ce que** l'extrait de *Quassia amara* est appliqué sur au moins une zone de peau et/ou muqueuses choisies parmi le cou, le décolleté, le ventre, les bras, les cuisses, les hanches, les fesses, la taille et/ou le visage, et préférentiellement le contour des yeux, les lèvres.

9. Utilisation selon l'une quelconque des revendications 1 à 8, **caractérisée en ce que** l'extrait de *Quassia amara* est dissous dans un solvant polaire choisi parmi l'eau, un alcool, un polyol, un glycol ou un de leurs mélanges.

10. Utilisation selon l'une quelconque des revendications 1 à 9, **caractérisé en ce que** l'extrait de Quassia amara est présent dans une composition cosmétique comprenant un excipient cosmétique topiquement acceptable.

11. Utilisation selon la revendication 10, **caractérisée en ce que** l'extrait de Quassia amara est présent dans la composition à une concentration de 1.10-4 % à 10%, préférentiellement de 1.10-3 % à 3 %, et encore préférentiellement de 1.10-2 % à 1% en poids, par rapport au poids total de la composition.

12. Utilisation selon l'une quelconque des revendications 10 ou 11 **caractérisée en ce que** la composition cosmétique contient également un ou plusieurs agents tenseurs et/ou un ou plusieurs agents antimicrobiens et/ou un ou plusieurs agents antiradicalaires et/ou un ou plusieurs agents apaisants et/ou un ou plusieurs agents amincissants et/ou un ou plusieurs agents actifs sur la microcirculation.

13. Utilisation selon l'une quelconque des revendications 10 à 12, **caractérisée en ce que** la composition cosmétique est choisie parmi une solution, aqueuse ou huileuse, une crème ou un gel aqueux ou un gel huileux, notamment un gel douche, un shampoing ; un lait ; une émulsion, une microémulsion ou une nanoémulsion, notamment huile-dans-eau ou eau-dans-huile ou multiple ou siliconée ; un masque ; un sérum ; une lotion ; un savon liquide ; un pain dermatologique ; une pommade ; une mousse ; un patch ; un produit anhydre, de préférence liquide, pâteux ou solide, par exemple sous forme de poudres de maquillage, de bâtonnet ou de stick, notamment sous forme de rouge à lèvre.

14. Procédé de soin cosmétique **caractérisé en ce qu'**il comprend l'application par voie topique sur au moins une zone de peau et/ou de muqueuses, d'un extrait de Quassia amara pour augmenter l'expression génique et/ou protéique du collagène et/ou la fermeté de la peau et/ou des muqueuses.

15. Procédé de soin cosmétique selon la revendication 14, **caractérisé en ce que** le collagène est le collagène de type I.

16. Procédé de soin cosmétique selon l'une quelconque des revendications 14 ou 15, **caractérisé en ce que** l'extrait de *Quassia amara* est tel que défini dans l'une des revendications 2, 3, 6 et 9 à 13.

17. Procédé de soin cosmétique selon l'une quelconque des revendications 14 à 16, **caractérisé en ce que** la zone de peau et/ou muqueuses est choisie parmi au moins le cou, le décolleté, le ventre, les bras, les cuisses, les hanches, les fesses, la taille et/ou le visage, et préférentiellement le contour des yeux, les lèvres.
